# EUROPEAN PATENT APPLICATION

(11) **EP 4 796 134 A1**
(43) Date of publication of application: **26.08.2026**
(21) Application number: 25890412.7
(22) Date of filing: 17.11.2025
(51) Int. Cl.: A61L 24/02, A61L 24/00, C08J 3/075, C08J 3/24, A61L 24/08, C08L 3/02

(54) **PREPARATION METHOD FOR STARCH HYDROGEL MICROSPHERES**

(30) Priority: 19.11.2024 CN 202411661882
(71) Applicant: Cardiolink Science (Shenzhen) Medical Technology Development Co., Ltd., Shenzhen, Guangdong 518100 (CN)
(72) Inventor: SUN, Hongtao, Shenzhen, Guangdong 518000 (CN); LIN, Yi, Shenzhen, Guangdong 518000 (CN); WANG, Fan, Shenzhen, Guangdong 518000 (CN); WANG, Yu, Shenzhen, Guangdong 518000 (CN); XUE, Jia, Shenzhen, Guangdong 518000 (CN); SUN, Peng, Shenzhen, Guangdong 518000 (CN); CHE, Haibo, Shenzhen, Guangdong 518000 (CN)
(74) Representative: Ghirardi, Valeria
(86) International application number: PCT/CN2025/135266
(87) International publication number: WO 2026/108747

(57) **Abstract**

The present application provides a preparation method for starch hydrogel microspheres, belonging to the technical field of embolic microsphere preparations. The preparation method for starch hydrogel microspheres includes following steps: adding a first crosslinking agent to a starch solution and mixing to obtain an aqueous-phase solution; adding an oil-phase solution to the aqueous-phase solution and mixing to obtain a mixed liquid; and adding a second crosslinking agent to the mixed liquid and mixing for reaction to obtain starch hydrogel microspheres. The starch hydrogel microspheres prepared by the method has advantages of long duration of *in vivo* degradation and adjustable duration of the degradation, and can be applied to a wide range of disease types.

## Description

### CROSS REFERENCE TO RELATED APPLICATION

The present application claims priority to Chinese Patent Application No. 202411661882.6, filed with the China National Intellectual Property Administration on November 19, 2024, and entitled "preparation method for starch hydrogel microspheres", which is incorporated herein by reference in entirety.

### TECHNICAL FIELD

The present application relates to the technical field of embolic microsphere preparations, specifically relates to a preparation method for starch hydrogel microspheres.

### BACKGROUND ART

Embolic materials are a type of material commonly used in the medical field, used to block blood or fluid in blood vessels or other organs. They coagulate into a solid-like substance in blood or liquid with which they come into contact, playing a role in blocking flow of liquid. Embolic materials generally possess good biocompatibility, bioabsorption, and other biological and physical properties to adapt to specific medical application environments thereof. Vascular embolization has a wide range of applications, it can be used for a treatment of vascular lesions such as vascular rupture and bleeding, arteriovenous malformations, aneurysms, and arteriovenous fistulas, as well as used for a treatment of diseases such as hemorrhagic tumors, tumor-like lesions, and hyperfunction of organs. In short, regardless of a type of lesion, as long as a clinical therapeutic goal can be achieved through vascular embolization without causing damage to important tissues and organs functions, and patients can tolerate postoperative reactions of embolization, vascular embolization can be considered for treatment.

Currently, the most widely used embolic materials in China are PVA microspheress and gelatin sponge particles, wherein, permanent implantation of PVA microspheress due to nonbiodegradability thereof may cause persistent inflammation and other side effects, which greatly affects willingness of patients to choose embolization. Gelatin sponge particles (Ailicon) are made from pigskin gelatin, are insoluble in water, but are biodegradable *in vivo*, serving as a medium-term embolic substance, with a duration of complete degradation of 14-90 days. Although gelatin sponge particles provide patients with an alternative biodegradable embolic agent, their animal-derived nature and the presence of formaldehyde cross-linking agents with toxic side effects make them unsuitable as a universally applicable option.

Based on this, given respective shortcomings of existing embolic materials, those skilled have considered using natural plant-derived starch for preparation of the embolic materials. However, starch embolic microspheress prepared by current processes suffer from excessively short *in vivo* degradation period (typically no more than 24 h), resulting in fewer applicable diseases.

### SUMMARY

An objective of the present application is to provide a preparation method for starch hydrogel microspheres. The starch hydrogel microspheres prepared by the method has advantages of long duration of *in vivo* degradation and adjustable duration of degradation, and can be applied to many disease types.

An embodiment of the present application is implemented as follows:
The embodiment of the present application provides a preparation method for starch hydrogel microspheres, including following steps:
adding a first crosslinking agent to a starch solution and mixing to obtain an aqueous-phase solution; adding an oil-phase solution to the aqueous-phase solution and mixing to obtain a mixed liquid; and adding a second crosslinking agent to the mixed liquid and mixing for reaction to obtain starch hydrogel microspheres.

In the above technical solution, during the preparation of the starch hydrogel microspheres, the cross-linking agent is added in two steps. Specifically, the first cross-linking agent is added during a process of forming the aqueous-phase solution, and the second crosslinking agent is added to the system after the aqueous phase and the oil phase are mixed. The crosslinking agent adopts the specific feeding method, allowing the resulting starch hydrogel microspheres to have a longer duration of the *in vivo* degradation, meanwhile the duration of the *in vivo* degradation of the resulting starch hydrogel microspheres can be adjusted by adjusting the mass ratio of the first crosslinking agent to the second cross-linking agent, thereby matching more disease types.

In some optional embodiments, a mass ratio of the first crosslinking agent to the second crosslinking agent is 1:(0.4-1.5).

In the above technical solution, the mass ratio of the first crosslinking agent to the second crosslinking agent is limited to a specific range, so that the duration of the *in vivo* degradation of the resulting starch hydrogel microspheres can be adjusted over a wider range, thus enabling to be applicable to more disease types.

In some optional embodiments, a mass of the first crosslinking agent is greater than a mass of the second crosslinking agent.

In the above technical solution, the mass of the first crosslinking agent is set to be greater than the mass of the second crosslinking agent, so that the duration of the *in vivo* degradation of the resulting starch hydrogel microspheres can be greater than 7 days, thus enabling to be particularly applicable to diseases that require long-term embolization.

In some optional embodiments, a mass of the first crosslinking agent is less than a mass of the second crosslinking agent.

In the above technical solution, the mass of the first crosslinking agent is set to be less than the mass of the second crosslinking agent, so that the duration of the *in vivo* degradation of the resulting starch hydrogel microspheres can be less than 7 days (such as, 2 to 5 days), thus enabling to be particularly applicable to diseases that require short-term embolization.

In some optional embodiments, the first crosslinking agent and/or the second crosslinking agent includes a phosphate crosslinking agent.

Optionally, the phosphate crosslinking agent includes sodium trimetaphosphate and/or sodium tripolyphosphate.

Optionally, the phosphate crosslinking agent is sodium trimetaphosphate.

Optionally, the phosphate crosslinking agent is sodium tripolyphosphate.

Optionally, the phosphate crosslinking agent is a mixture of sodium trimetaphosphate (STMP) and sodium tripolyphosphate (STPP).

In the above technical solution, the first crosslinking agent and/or the second crosslinking agent use phosphate crosslinking agents. Due to low toxicity of the type of the crosslinking agent, it can be well adapted to natural plant-based starch, so that the resulting starch hydrogel microspheres have good biocompatibility.

Further, using a mixture of sodium trimetaphosphate and sodium tripolyphosphate as the phosphate crosslinking agent has an advantage of better crosslinking effect.

In some optional embodiments, in the mixture, a mass ratio of the sodium trimetaphosphate to the sodium tripolyphosphate is 10:(0.8-1.2).

In the above technical solution, the mass ratio of the two crosslinking agents in the mixture is limited within a specific range, so that a more excellent crosslinking effect can be achieved.

In some optional embodiments, in the aqueous-phase solution, a mass ratio of starch to the first crosslinking agent is 10:(1-2).

In the above technical solution, the mass ratio of the starch to the first crosslinking agent in the aqueous-phase solution is limited within a specific range, so that the two have a more suitable mass proportion in the aqueous phase, thereby enabling the resulting starch hydrogel microspheres to have advantages of good sphericity and resistance to aggregation.

In some optional embodiments, in the starch solution, a mass concentration of starch is 8 g/mL-12 g/mL.

In the above technical solution, the mass concentration of starch in the starch solution is limited within a specific range. The starch solution with an appropriate concentration can be better mixed with the crosslinking agent and dispersed, thereby enabling the resulting starch hydrogel microspheres to have advantages of good sphericity and resistance to aggregation.

In some optional embodiments, a preparation step for the oil-phase solution includes: adding an emulsifier to a mixed solvent of cyclohexane and n-decane and mixing to obtain an oil-phase solution.

In the above technical solution, during the preparation process of the oil-phase solution, using a mixed system of cyclohexane and n-octane as a solvent can dissolve and disperse the emulsifier well, thereby achieving a better emulsification effect after subsequent mixing of the oil phase and the water phase.

In some optional embodiments, in the mixed solvent, a volume ratio of the cyclohexane to the n-decane is 1:(0.8-1.2).

In the above technical solution, the volume ratio of the cyclohexane to the n-decane in the mixed solvent is limited within a specific range, so that the two have a more appropriate volume ratio in the miscible system, thereby better dissolving and dispersing the emulsifier, thereby achieving a better emulsification effect after subsequent mixing of the oil phase and the water phase.

### BRIEF DESCRIPTION OF DRAWINGS

To more clearly illustrate the technical solutions of embodiments of the present application, drawings need to be used in the embodiments will be briefly described below. It should be understood that the drawings below only show some embodiments of the present application and should not be considered as a limitation to the scope.
FIG. 1 is a process flow diagram of a preparation method for starch hydrogel microspheres provided by an embodiment of the present application;
FIG. 2 is an optical microscope image of the starch hydrogel microspheres provided in Example 1 of the present application;
FIG. 3 is an optical microscope image of the starch hydrogel microspheres provided in Example 2 of the present application;
FIG. 4 is an optical microscope image of the starch hydrogel microspheres provided in Comparative Example 1 of the present application;
FIG. 5 is an optical microscope image of the starch hydrogel microspheres provided in Comparative Example 2 of the present application;
FIG. 6 is an optical microscope image of the starch hydrogel microspheres provided in Comparative Example 3 of the present application;
FIG. 7 is a test result of the *in vivo* degradation provided in Control Group 1 of the present application;
FIG. 8 is a test result of the *in vivo* degradation provided in Control Group 2 of the present application;
FIG. 9 is a test result of the *in vivo* degradation provided in Example 1 of the present application;
FIG. 10 is a test result of the *in vivo* degradation provided in Example 2 of the present application; and
FIG. 11 is a test result of the *in vivo* degradation provided in Comparative Example 1 of the present application.

### DETAILED DESCRIPTION OF EMBODIMENTS

To make objectives, technical solutions, and advantages of embodiments of the present application clearer, the technical solutions in the embodiments of the present application will be clearly and completely described below. Specific conditions are not specified in the embodiments, conventional conditions or conditions recommended by the manufacturer shall be followed. Reagents or instruments used without specified manufacturers are all regular products that can be purchased through the market.

The following is a detailed description of the preparation method for starch hydrogel microspheres according to embodiments of the present application.

The embodiments of the present application provide a preparation method for starch hydrogel microspheres, including following steps:
adding a first crosslinking agent to a starch solution and mixing to obtain an aqueous-phase solution; adding an oil-phase solution to the aqueous-phase solution and mixing to obtain a mixed liquid; and adding a second crosslinking agent to the mixed liquid and mixing for reaction to obtain starch hydrogel microspheres.

In the present application, during a process of the preparation of the starch hydrogel microspheres, the cross-linking agent is added in two steps. Specifically, the first cross-linking agent is added during a process of forming the aqueous-phase solution, and the second crosslinking agent is added to the system after the aqueous phase and the oil phase are mixed. The crosslinking agent adopts the specific gradient feeding method, allowing the resulting starch hydrogel microspheres to have a relatively long duration of the *in vivo* degradation, meanwhile the duration of the *in vivo* degradation of the resulting starch hydrogel microspheres can be adjusted by adjusting the mass ratio of the first crosslinking agent to the second cross-linking agent, thereby matching more disease types.

It should be noted that "the crosslinking agent adopts the specific gradient feeding method, allowing the resulting starch hydrogel microspheres to have a relatively long duration of the *in vivo* degradation", wherein "a relatively long duration of the degradation" is compared with existing starch hydrogel microspheress with a duration of *in vivo* degradation of less than 24 h.

It should be noted that a preparation process of the starch solution can be performed according to conventional methods in the art, for example, adding starch and sodium hydroxide to distilled water and stirring under a condition of heating until a transparent paste is formed.

It should be noted that methods of adding the first crosslinking agent and/or the second crosslinking agent are not limited; for example, they can be in a solid form or in a liquid form. In the embodiments of the present application, they are all added in a liquid form, such as, in a form of an aqueous solution of 0.2 g/mL-0.25 g/mL.

It is understood that after the mixing reaction step, steps of settling, filtering, washing, and sieving are included in sequence, and each step can be performed according to conventional operations in the art.

It should be noted that the mass ratio of the first crosslinking agent to the second crosslinking agent is not limited and can be adjusted adaptively according to actual needs.

As an example, the mass ratio of the first crosslinking agent to the second crosslinking agent is 1:(0.4-1.5), such as, but is not limited to any one value of the following mass ratios of 1:0.4, 1:0.5, 1:0.6, 1:0.7, 1:0.8, 1:0.9, 1:1, 1:1.1, 1:1.2, 1:1.3, 1:1.4, and 1:1.5, or a range value between any two thereof.

In the present embodiment, the mass ratio of the first crosslinking agent to the second crosslinking agent is limited to a specific range, so that the duration of the *in vivo* degradation of the resulting starch hydrogel microspheres can be adjusted over a wider range, thus enabling to be applicable to more disease types.

As an example, a mass of the first crosslinking agent is greater than a mass of the second crosslinking agent.

In the present embodiment, the mass of the first crosslinking agent is set to be greater than the mass of the second crosslinking agent, so that the duration of the *in vivo* degradation of the resulting starch hydrogel microspheres can be greater than 7 days, thus enabling to be particularly applicable to diseases that require long-term embolization.

As an example, the mass of the first crosslinking agent is less than the mass of the second crosslinking agent.

In the present embodiment, the mass of the first crosslinking agent is set to be less than the mass of the second crosslinking agent, so that the duration of the *in vivo* degradation of the resulting starch hydrogel microspheres can be less than 7 days (such as, 2 to 5 days), thus enabling to be particularly applicable to diseases that require short-term embolization.

It should be noted that the type of the first crosslinking agent and/or the second crosslinking agent is not limited and can be adjusted adaptively according to actual needs.

As an example, the first crosslinking agent and/or the second crosslinking agent includes a phosphate crosslinking agent.

As an example, the phosphate crosslinking agent includes sodium trimetaphosphate and/or sodium tripolyphosphate.

As an example, the phosphate crosslinking agent is sodium trimetaphosphate.

As an example, the phosphate crosslinking agent is sodium tripolyphosphate.

As an example, the phosphate crosslinking agent is a mixture of sodium trimetaphosphate and sodium tripolyphosphate.

In the present embodiment, the first crosslinking agent and/or the second crosslinking agent use a phosphate crosslinking agent. Due to low toxicity of the type of the crosslinking agent, it can be well adapted to natural plant-based starch, so that the resulting starch hydrogel microspheres have good biocompatibility.

In the present embodiment, using a mixture of sodium trimetaphosphate and sodium tripolyphosphate as the phosphate crosslinking agent has an advantage of better crosslinking effect.

As an example, in the mixture, a mass ratio of the sodium trimetaphosphate to the sodium tripolyphosphate is 10:(0.8-1.2), such as, but is not limited to any one value of the following mass ratios of 10:0.8, 10:0.9, 10:1, 10:1.1, and 10:1.2, or a range value between any two thereof.

In the present embodiment, the mass ratio of the two crosslinking agents in the mixture is limited within a specific range, so that a more excellent crosslinking effect can be achieved.

As an example, in the aqueous-phase solution, a mass ratio of the starch to the first crosslinking agent is 10: (1-2), such as, but is not limited to any one of the following mass ratios of 10:1, 10:1.1, 10:1.2, 10:1.3, 10:1.4, 10:1.5, 10:1.6, 10:1.7, 10:1.8, 10:1.9 and 10, or a range value between any two thereof.

In the present embodiment, the mass ratio of the starch to the first crosslinking agent in the aqueous-phase solution is limited within a specific range, so that the two have a suitable mass proportion in the aqueous phase, thereby enabling the resulting starch hydrogel microspheres to have advantages of good sphericity and resistance to aggregation.

As an example, in the starch solution, a mass concentration of the starch is 8 g/mL-12 g/mL, such as, but is not limited to any one value of the following mass concentrations of 8 g/mL, 9 g/mL, 10 g/mL, 11 g/mL, and 12 g/mL, or a range value between any two thereof.

In the present embodiment, a mass concentration of the starch in the starch solution is limited within a specific range. The starch solution with an appropriate concentration can be better mixed with the crosslinking agent and dispersed, thereby enabling the resulting starch hydrogel microspheres to have advantages of good sphericity and resistance to aggregation.

As an example, a preparation step for the oil-phase solution includes: adding an emulsifier to a mixed solvent of cyclohexane and n-decane and mixing to obtain an oil-phase solution.

In the present embodiment, during a preparation process of the oil-phase solution, using a mixed system of cyclohexane and n-octane as a solvent can dissolve and disperse the emulsifier well, thereby achieving a better emulsification effect after subsequent mixing of the oil phase and the water phase.

As an example, in the mixed solvent, a volume ratio of the cyclohexane to the n-decane is 1:(0.8-1.2), such as, but is not limited to any one value of the following mass ratios of 1:0.8, 1:0.9, 1:1, 1:1.1, and 1:2, or a range value between any two thereof.

In the present embodiment, the volume ratio of the cyclohexane to the n-decane in the mixed solvent is mixed within a specific range, so that for the two have a more appropriate volume ratio in the miscible system, thereby better dissolving and dispersing the emulsifier, thereby achieving a better emulsification effect after subsequent mixing of the oil phase and the water phase.

It should be noted that the type of the emulsifier is not limited and can be selected and set according to conventional practices in the art. For example, the emulsifier can be OP-4.

It should be noted that for the starch hydrogel microspheress, processes or steps that are not specifically specified or defined can all be set according to conventional practices in the art.

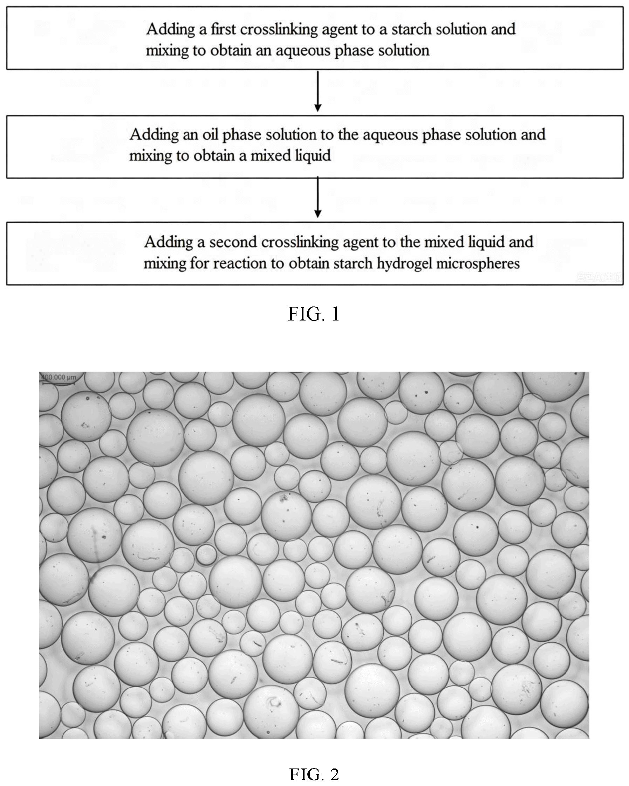

As an example, a process flow diagram of a preparation method for starch hydrogel microspheres is shown in FIG. 1 for example.

Features and performances of the present application will be further described in detail below in combination with the examples.

### Example 1

The present example of the present application provided a preparation method for starch hydrogel microspheres, including following steps:
10 g of starch and 1 g of sodium hydroxide were added to 100 mL of distilled water, heated and stirred until a transparent paste was formed. The paste was then allowed to stand and cool for later use. 25 mL of the starch solution was taken, and 7 mL of an aqueous solution containing 1.4 g of STMP and 0.14 g of STPP was added to the starch solution under a condition of stirring. The mixture was stirred until fully dissolved to obtain an aqueous-phase solution. 0.5 g of emulsifier (OP-4) was added to 100 mL of a mixed solvent (cyclohexane and n-decane in a volume ratio of 1:1) and stirred to obtain an oil-phase solution. The oil-phase solution was added to the aqueous-phase solution and stirred to mix to obtain a mixed liquid. 3 mL of an aqueous solution containing 0.6 g of STMP and 0.06 g of STPP was added to the mixed liquid. The mixture was stirred at room temperature (25 °C) for 24 h. After the reaction was completed, treatments of settling, filtration, washing (wash three times with ethyl acetate to remove oil phase impurities), and sieving was sequentially performed to obtain starch hydrogel microspheres.

### Example 2

The present example of the present application provided a preparation method for starch hydrogel microspheres, including following steps:
10 g of starch and 1 g of sodium hydroxide were added to 100 mL of distilled water, heated and stirred until a transparent paste was formed. The paste was then allowed to stand and cool for later use. 25 mL of the starch solution was taken, and 7 mL of an aqueous solution containing 1.4 g of STMP and 0.14 g of STPP was added to the starch solution under a condition of stirring. The mixture was stirred until fully dissolved to obtain an aqueous-phase solution. 0.5 g of emulsifier (OP-4) was added to 100 mL of a mixed solvent (cyclohexane and n-decane in a volume ratio of 1:1) and stirred to obtain an oil-phase solution. The oil-phase solution was added to the aqueous-phase solution and stirred to mix to obtain a mixed liquid. 10 mL of an aqueous solution containing 2 g of STMP and 0.2 g of STPP was added to the mixed liquid. The mixture was stirred at room temperature (25 °C) for 24 h. After the reaction was completed, treatments of settling, filtration, washing (wash three times with ethyl acetate to remove oil phase impurities), and sieving was sequentially performed to obtain starch hydrogel microspheres.

### Example 3

The present example of the present application provided a preparation method for starch hydrogel microspheres, including following steps:
10 g of starch and 1 g of sodium hydroxide were added to 100 mL of distilled water, heated and stirred until a transparent paste was formed. The paste was then allowed to stand and cool for later use. 25 mL of the starch solution was taken, and 7 mL of an aqueous solution containing 1.4 g of STMP and 0.14 g of STPP was added to the starch solution under a condition of stirring. The mixture was stirred until fully dissolved to obtain an aqueous-phase solution. 0.5 g of emulsifier (OP-4) was added to 100 mL of a mixed solvent (cyclohexane and n-decane in a volume ratio of 1:1) and stirred to obtain an oil-phase solution. The oil-phase solution was added to the aqueous-phase solution and stirred to mix to obtain a mixed liquid. 5 mL of an aqueous solution containing 1 g of STMP and 0.1 g of STPP was added to the mixed liquid. The mixture was stirred at room temperature (25 °C) for 24 h. After the reaction was completed, treatments of settling, filtration, washing (wash three times with ethyl acetate to remove oil phase impurities), and sieving was sequentially performed to obtain starch hydrogel microspheres.

### Example 4

The present example of the present application provided a preparation method for starch hydrogel microspheres, including following steps:
10 g of starch and 1 g of sodium hydroxide were added to 100 mL of distilled water, heated and stirred until a transparent paste was formed. The paste was then allowed to stand and cool for later use. 25 mL of the starch solution was taken, and 7 mL of an aqueous solution containing 1.5 g of STMP was added to the starch solution under a condition of stirring. The mixture was stirred until fully dissolved to obtain an aqueous-phase solution. 0.5 g of emulsifier (OP-4) was added to 100 mL of a mixed solvent (cyclohexane and n-decane in a volume ratio of 1:1) and stirred to obtain an oil-phase solution. The oil-phase solution was added to the aqueous-phase solution and stirred to mix to obtain a mixed liquid. 5 mL of an aqueous solution containing 1 g of STMP was added to the mixed liquid. The mixture was stirred at room temperature (25 °C) for 24 h. After the reaction was completed, treatments of settling, filtration, washing (wash three times with ethyl acetate to remove oil phase impurities), and sieving was sequentially performed to obtain starch hydrogel microspheres.

### Comparative Example 1

The comparative example of the present application provided a preparation method for starch hydrogel microspheres, including following steps:
10 g of starch and 1 g of sodium hydroxide were added to 100 mL of distilled water, heated and stirred until a transparent paste was formed. The paste was then allowed to stand and cool for later use. 25 mL of the starch solution was taken, and 7 mL of an aqueous solution containing 1.4 g of STMP and 0.14 g of STPP was added to the starch solution under a condition of stirring. The mixture was stirred until fully dissolved to obtain an aqueous-phase solution. 0.5 g of emulsifier (OP-4) was added to 100 mL of a mixed solvent (cyclohexane and n-decane in a volume ratio of 1:1) and stirred to obtain an oil-phase solution. The oil-phase solution was added to the aqueous-phase solution, stirred and reacted at room temperature (25 °C) for 24 h. After the reaction was completed, treatments of settling, filtration, washing (wash three times with ethyl acetate to remove oil phase impurities), and sieving was sequentially performed to obtain starch hydrogel microspheres.

### Comparative Example 2

The comparative example of the present application provided a preparation method for starch hydrogel microspheres, including following steps:
10 g of starch and 1 g of sodium hydroxide were added to 100 mL of distilled water, heated and stirred until a transparent paste was formed. The paste was then allowed to stand and cool for later use. 0.5 g of emulsifier (OP-4) was added to 100 mL of a mixed solvent (cyclohexane and n-decane in a volume ratio of 1:1) and stirred to obtain an oil-phase solution. 25 mL of the starch solution was taken, and the oil-phase solution was added to the starch solution under a condition of stirring, and stirred to mix to obtain a mixed liquid. 7 mL of an aqueous solution containing 1.4 g of STMP and 0.14 g of STPP was then added to the mixed liquid. The mixture was stirred and reacted at room temperature (25 °C) for 24 h. After the reaction was completed, treatments of settling, filtration, washing (wash three times with ethyl acetate to remove oil phase impurities), and sieving was sequentially performed to obtain starch hydrogel microspheres.

### Comparative Example 3

The comparative example of the present application provided a preparation method for starch hydrogel microspheres, including following steps:
10 g of starch and 1 g of sodium hydroxide were added to 100 mL of distilled water, heated and stirred until a transparent paste was formed. The paste was allowed to stand and cool for later use. 0.5 g of emulsifier (OP-4) was added to 100 mL of a mixed solvent (cyclohexane and n-decane in a volume ratio of 1:1) and stirred to obtain an oil-phase solution. 25 mL of the starch solution was taken, and the oil-phase solution was added to the starch solution under a condition of stirring, and stirred to mix to obtain a mixed liquid. 17 mL of an aqueous solution containing 3.4 g of STMP and 0.34 g of STPP was then added to the mixed liquid. The mixture was stirred at room temperature (25 °C) for 24 h. After the reaction was completed, treatments of settling, filtration, washing (wash three times with ethyl acetate to remove oil phase impurities), and sieving was sequentially performed to obtain starch hydrogel microspheres.

### Test Example

### 1. Microstructure test of hydrogel microspheres

### Test method

The starch hydrogel microspheress prepared in Examples 1-2 and Comparative Examples 1-3 were numbered, respectively, and then the microstructure of each sample was tested, respectively.

Specific test steps were as follows:
100 mL of microspheress (300 µm-600 µm) after sieving in each group was added to 100 mL of physiological saline, respectively. After standing and allowing the microspheress to settle and separate into layers, the supernatant was removed, and 100 mL of physiological saline was added again. The process was repeated at least three times to obtain replacement microspheress with physiological saline as a preservation medium. The microstructure of the microspheress was observed by photographing using an optical microscope.

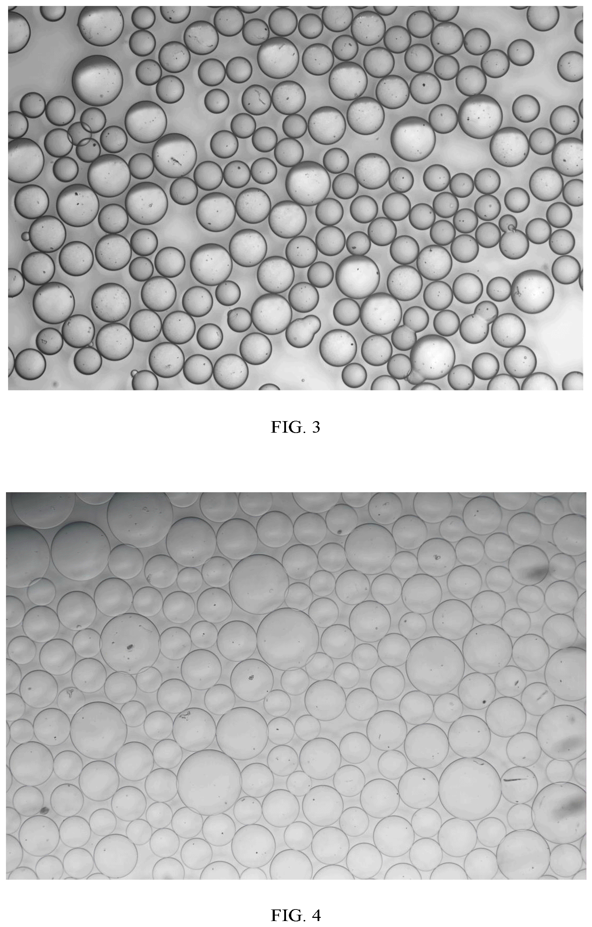

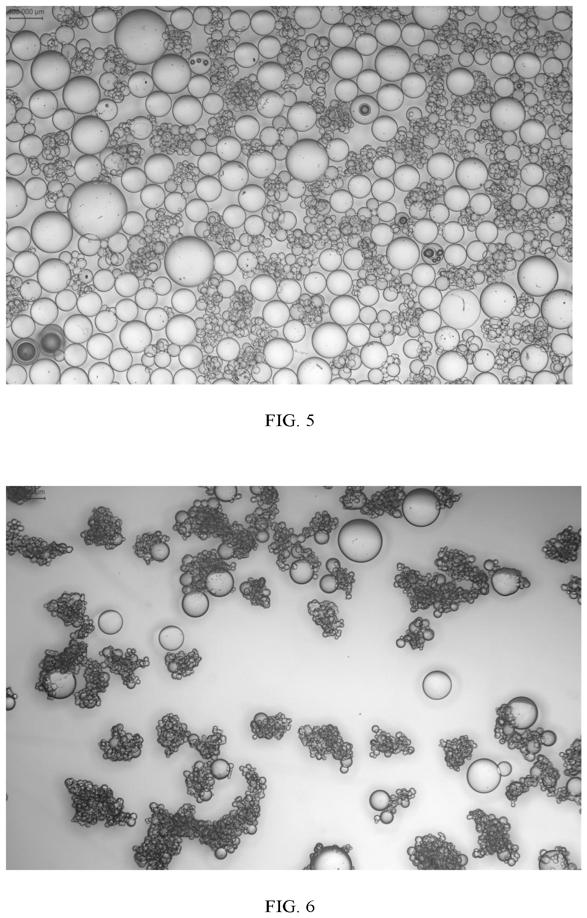

Refer to FIG. 2- FIG. 6, wherein FIG. 2 corresponds to Example 1 (scale bar 400 µm), FIG. 3 corresponds to Example 2 (scale bar 400 µm), FIG. 4 corresponds to Comparative Example 1 (scale bar 400 µm), FIG. 5 corresponds to Comparative Example 2 (scale bar 400 µm), and FIG. 6 corresponds to Comparative Example 3 (scale bar 300 µm).

As shown in FIG. 2- FIG. 6, the starch hydrogel microspheress prepared according to the preparation method provided in Examples of the present application exhibit advantages of good sphericity and dispersibility. It should be particularly noted that if the crosslinking agent is added only after mixing the aqueous phase and the oil phase, the resulting microspheress will agglomerate severely, making subsequent applications difficult. Moreover, the agglomeration of the microspheress becomes more severe with increasing amounts of crosslinking agent.

### 2. Solid Content Test of hydrogel microspheres

### Test method

The starch hydrogel microspheress prepared in Examples 1-4 and Comparative Examples 1-3 were numbered, respectively, and then the solid content of each sample was measured, respectively.

Specific test steps were as follows:
Water content of the microspheress (300-600 micrometers) before replacement in each group was measured using a moisture analyzer. The test was repeated three times, and an average value was calculated to obtain a corresponding solid content. Corresponding test results was summarized in Table 1.

### 3. In Vitro Degradation Duration Test of hydrogel microspheres

### Test method

The starch hydrogel microspheress prepared in Examples 1-4 and Comparative Examples 1-3 were numbered, respectively, and then the duration of *in vitro* degradation of each sample was measured, respectively.

Specific test steps were as follows:
2 mL of microspheress (300-600 micrometers) after replacement in each group was added to 18 mL of physiological saline solution containing α-amylase, preparing an enzyme concentration of 500 U/L. The mixture was incubated on a shaker at 37 °C. The reaction flask was visually observed at regular intervals. Once the microspheress were no longer visible, 0.5 mL of the mixture was diluted to 1 mL for microscopic photography. A duration of complete degradation of the microspheress was defined as the duration at which no microspheress are observed under microscopic photography. Corresponding test results was summarized in Table 1.

### 4. In Vivo Degradation Duration Test of hydrogel microspheres

### Test method

The starch hydrogel microspheress prepared in Examples 1-4 and Comparative Example 1 were numbered, respectively. Commercially available PVA microspheress (Creek (Shenzhen) Medical Technology Development Co., Ltd. - non-degradable) and gelatin sponge particles (Alicon Pharmaceutical Technology Co., Ltd., duration of complete degradation of 14-90 days) were used as control group 1 and control group 2. Then duration of *in vivo* degradation of each sample was then measured, respectively, and test results was summarized in Table 1.

Specific test steps were as follows:
Five healthy experimental rabbits were prepared. Vascular networks of the rabbit ears were photographed under a light source (i.e., preoperative photographs of vascular networks of the rabbit ears were collected). Then, a 22 G indwelling needle was inserted into a central artery 4-5 cm from a base of the ear, from a proximal end to a distal end, following a direction of blood flow. The needle was removed, and suspended sample was slowly injected (a pulsed injection at a rate of 1 mL/min) until significant injection resistance was felt. When light source illuminated from behind the ear, the central artery of the rabbit ear appeared white, with no blood flow inside. After the injection was completed, the injection site was pressed to stop bleeding, and the indwelling needle was simultaneously pulled out. The needle insertion site during the operation was marked. Then, the vascular network of the rabbit ears was photographed under a light source at 0 h after operation, 2 h after operation, 1 day after operation, 2 days after operation, and 7 days after operation, respectively, to statistically analyze the degradation status of the sample in the animal body.

It should be noted that if the vascular networks in the rabbit ears are completely normal, indicating that the samples have been completely degraded; if the vascular networks in the rabbit ears are still damaged, indicating that the samples have not been completely degraded, and the more severe the damage is, the better the embolization effect of the sample is (i.e., the more difficult it is to degrade).

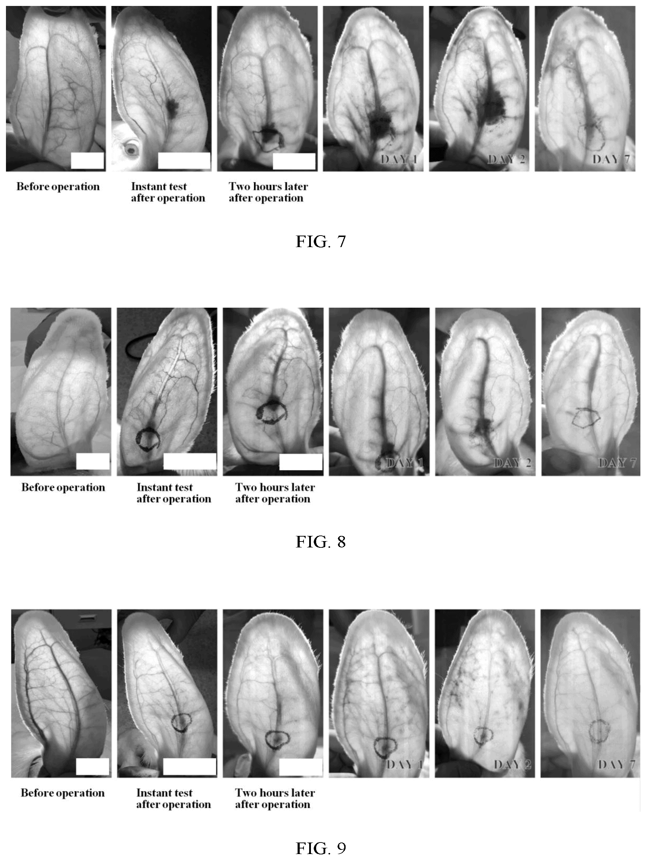

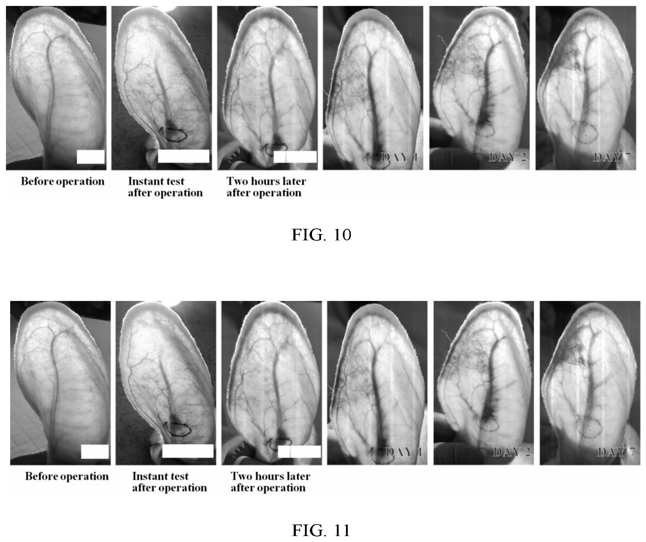

Refer to FIG. 7- FIG. 11, wherein FIG. 7 corresponds to Control Group 1, FIG. 8 corresponds to Control Group 2, FIG. 9 corresponds to Example 1, FIG. 10 corresponds to Example 2, and FIG. 11 corresponds to Comparative Example 1.

From test results in FIG. 7- FIG. 11, it can be seen that in Control Group 1 and Control Group 2, the vascular networks in the rabbit ears still had relatively severe damage 7 days after operation, indicating that the blood vessels were still in a severely blocked state (i.e., the samples had not decomposed). In Example 1, the vascular network in the rabbit ear recovered to normal between 2 and 7 days after operation, indicating that the duration of the *in vivo* degradation of the sample in Example 1 is 2-7 days. In Example 2, the damage to the vascular network of the rabbit ear was somewhat alleviated 7 days after operation compared to 1 day after operation, but it had not recovered to normal, indicating that the duration of the *in vivo* degradation of the sample in Example 2 is greater than 7 days. In Comparative Example 1, the vascular network in the rabbit ear recovered to normal 1 day after operation, indicating that the duration of the *in vivo* degradation of the sample in Comparative Example 1 is within 24 h.

**Table 1**

| Sample | Solid Content(%) | Duration of *In Vitro* Degradation | Duration of *In vivo* Degradation |
|---|---|---|---|
| Example 1 | 7.42 | 6 h | 2 h -24 h |
| Example 2 | 10.21 | 5 days | 2-7 days |
| Example 3 | 8.73 | 3 days | 1-5 days |
| Example 4 | 9.21 | 4 days | 1-5 days |
| Comparative Example 1 | 2.45 | >30 days | >7 days |
| Comparative Example 2 | 8.41 | - | - |
| Comparative Example 3 | 12.42 | - | - |
| Control Group 1 | - | - | >7 days |
| Control Group 2 | - | - | >7 days |

It should be noted that the "-" in Table 1 indicates that no test was performed.

Based on the test results in Table 1 and FIG. 2-FIG. 11, it can be seen that the resulting starch hydrogel microspheress prepared according to the preparation method provided in Examples of the present application have advantages of good sphericity and good dispersibility, and exhibit a long duration of the *in vivo* degradation and an adjustable specific duration of the degradation, making them applicable to a wide range of diseases.

The above embodiments are only some embodiments of the present application, not all of the embodiments of the present application. The detailed description of the embodiments of the present application is not intended to limit the scope of protection of the present application, but merely represents selected embodiments of the present application. Based on the embodiments of the present application, all other embodiments obtained by those skilled in the art without making inventive efforts are within the scope of protection of the present application.

## Claims

1. A preparation method for starch hydrogel microspheres, **characterized in that**, the preparation method comprises following steps:
adding a first crosslinking agent to a starch solution and mixing to obtain an aqueous-phase solution;
adding an oil-phase solution to the aqueous-phase solution and mixing to obtain a mixed liquid; and
adding a second crosslinking agent to the mixed liquid and mixing for reaction to obtain starch hydrogel microspheres.

2. The preparation method for starch hydrogel microspheres according to claim 1, **characterized in that**, a mass ratio of the first crosslinking agent to the second crosslinking agent is 1:(0.4-1.5).

3. The preparation method for starch hydrogel microspheres according to claim 2, **characterized in that**, a mass of the first crosslinking agent is greater than a mass of the second crosslinking agent.

4. The preparation method for starch hydrogel microspheres according to claim 2, **characterized in that**, a mass of the first crosslinking agent is less than a mass of the second crosslinking agent.

5. The preparation method for starch hydrogel microspheres according to any one of claims 1 to 4, **characterized in that**, the first crosslinking agent and/or the second crosslinking agent comprises a phosphate crosslinking agent;
optionally, the phosphate crosslinking agent comprises sodium trimetaphosphate and/or sodium tripolyphosphate;
optionally, the phosphate crosslinking agent is sodium trimetaphosphate;
optionally, the phosphate crosslinking agent is sodium tripolyphosphate; and
optionally, the phosphate crosslinking agent is a mixture of sodium trimetaphosphate and sodium tripolyphosphate.

6. The preparation method for starch hydrogel microspheres according to claim 5, **characterized in that**, in the mixture, a mass ratio of the sodium trimetaphosphate to the sodium tripolyphosphate is 10:(0.8-1.2).

7. The preparation method for starch hydrogel microspheres according to any one of claims 1 to 4, **characterized in that**, in the aqueous-phase solution, a mass ratio of the starch to the first crosslinking agent is 10:(1-2).

8. The preparation method for starch hydrogel microspheres according to any one of claims 1 to 4, **characterized in that**, in the starch solution, a mass concentration of starch is 8 g/mL-12 g/mL.

9. The preparation method for starch hydrogel microspheres according to any one of claims 1 to 4, **characterized in that**, preparation for the oil-phase solution comprises: adding an emulsifier to a mixed solvent of cyclohexane and n-decane and mixing to obtain the oil-phase solution.

10. The preparation method for starch hydrogel microspheres according to claim 9, **characterized in that**, in the mixed solvent, a volume ratio of the cyclohexane to the n-decane is 1:(0.8-1.2).
